(19) Europäisches Patentamt
European Patent Office
Office européen des brevets

(11) Veröffentlichungsnummer : **0 041 131**
**B1**

(12) # EUROPÄISCHE PATENTSCHRIFT

(45) Veröffentlichungstag der Patentschrift : **13.06.84**

(51) Int. Cl.³ : **C 07 D317/46**, C 07 D319/20

(21) Anmeldenummer : **81103261.4**

(22) Anmeldetag : **25.04.79**

(60) Veröffentlichungsnummer der früheren Anmeldung nach Art. 76 EPÜ : **0006978**

(54) **Herstellung fluorsubstituierter Benzodioxole und Benzodioxane.**

(30) Priorität : **05.05.78 DE 2819788**

(43) Veröffentlichungstag der Anmeldung : **09.12.81 Patentblatt 81/49**

(45) Bekanntmachung des Hinweises auf die Patenterteilung : **13.06.84 Patentblatt 84/24**

(84) Benannte Vertragsstaaten : **BE CH DE FR GB IT NL**

(56) Entgegenhaltungen :
**US-A- 3 749 677**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Lantzsch, Reinhard, Dr.**
**Heymannstrasse 32**
**D-5090 Leverkusen (DE)**
Erfinder : **Marhold, Albrecht, Dr.**
**Carl-Duisberg-Strasse 329**
**D-5090 Leverkusen (DE)**
Erfinder : **Behrenz, Wolfgang, Dr.**
**Untergründemich 14**
**D-5063 Overath (DE)**
Erfinder : **Hammann, Ingeborg, Dr.**
**Belfordstrasse 9**
**D-5000 Köln (DE)**

EP 0 041 131 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Herstellung fluorsubstituierter Benzodioxole und Benzodioxane sowie neue fluorsubstituierte Benzodioxane.

Es wurde ein Verfahren zur Herstellung der Verbindungen der Formel XIV gefunden,

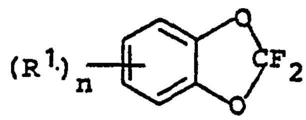 (XIV)

in welcher

n für 1 bis 4 steht und

$R^1$ für gleiche oder verschiedene Reste aus der Gruppe Wasserstoff, $C_1$-$C_6$-Alkyl, Trihalogenmethyl, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, ferner für CN, $-CH-Hal_2$, $-CHO$, Halogen, gegebenenfalls substituiertes Phenyl oder Phenoxy, Chlorcarbonyl, Chlorsulfonyl oder Nitro steht oder zwei benachbarte Reste gemeinsam mit den angrenzenden C-Atomen einen gegebenenfalls substituierten ankondensierten Benzolring bilden, und

$R^2$ für Wasserstoff, $C_{1-4}$-Alkyl, CN, $-C \equiv CH$ steht und

Hal für Halogen steht,

das dadurch gekennzeichnet ist, daß man Verbindungen der allgemeinen Formel XV

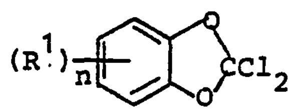 (XV)

in welcher

$R^1$ und n die oben angegebene Bedeutung haben,

mit wasserfreier Flußsäure umsetzt.

Es wurde ferner ein Verfahren zur Herstellung der Verbindungen der allgemeinen Formel XVI gefunden,

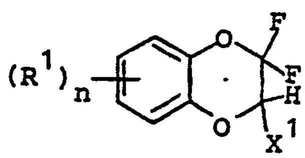 (XVI)

in welcher

n für 1 bis 4 steht und

$R^1$ für gleiche oder verschiedene Reste aus der Gruppe Wasserstoff, $C_{1-6}$-Alkyl, $C_{1-6}$-Fluoralkyl mit 3-6 Fluoratomen, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, $C_{2-6}$-Alkenyl, $C_{2-6}$-Alkinyl, Halogen, gegebenenfalls substituiertes Phenyl oder Phenoxy, Carbonyl, Nitro, Cyan, $SO_3H$ sowie für

$$-\underset{R^2}{\underset{|}{CH}}-OH,\ -CHO$$

steht oder zwei der Reste einen ankondensierten Benzolring bedeuten und

$X^1$ für Wasserstoff oder Halogen steht,

$R^2$ für Wasserstoff, CN, $C_{1-4}$-Alkyl oder $-C \equiv CH$ steht und

Hal für Halogen steht,

das dadurch gekennzeichnet ist, daß man Verbindungen der allgemeinen Formel (XVII)

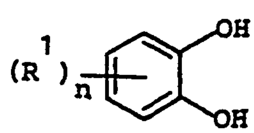 (XVII)

in welcher

$R^1$ und n die oben angegebene Bedeutung haben,

mit Verbindungen der allgemeinen Formel XVIII

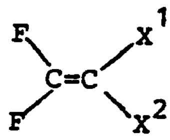 (XVIII)

in welcher
$X^2$ für Halogen steht, und
$X^1$ für Wasserstoff oder Halogen steht,
umsetzt.

Es wurden ferner die neuen Verbindungen der Formel XVI gefunden

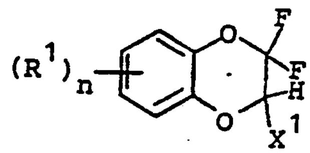 (XVI)

in welcher
n für 1 bis 4 steht und
$R^1$ für gleiche oder verschiedene Reste aus der Gruppe Wasserstoff, $C_{1-6}$-Alkyl, $C_{1-6}$-Fluoralkyl mit 3-6 Fluoratomen, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, $C_{2-6}$-Alkenyl, $C_{2-6}$-Alkinyl, Halogen, gegebenenfalls substituiertes Phenyl oder Phenoxy, Carbonyl, Nitro, Cyan, $SO_3H$ steht oder zwei der Reste einen ankondensierten Benzolring bedeuten und
$X^1$ für Wasserstoff oder Halogen steht.

1. Die Verbindungen der Formel XIV und XVI dienen zur Herstellung von neuen Benzylestern der Formel I

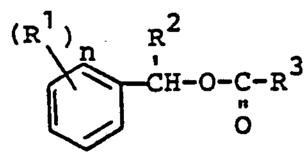 (I)

in welcher
n für 1-5 steht und
$R^1$ für gleiche oder verschiedene Reste aus der Gruppe Wasserstoff, $C_{1-6}$-Alkyl, $C_{1-6}$-Halogenalkyl mit 3-6 Halogenatomen, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, Halogen, gegebenenfalls substituiertes Phenyl, gegebenenfalls substituiertes Phenoxy, steht oder zwei benachbarte Reste gemeinsam mit den angrenzenden C-Atomen einen gegebenenfalls substituierten ankondensierten Benzolring oder ein oder mehrfach durch Fluor substituierte Sauerstoff enthaltende heterocyclische Fünf- oder Sechsringe bilden,
Dabei müßen in Formel I zwei benachbarte Reste gemeinsam mit den angrenzenden C-Atomen ein oder mehrfach durch Fluor substituierte Sauerstoff enthaltende heterocyclische Fünf- oder Sechsringe bilden,
$R^2$ für Wasserstoff, $C_{1-4}$-Alkyl, Cyan oder Ethinyl steht,
$R^3$ für Reste der Formel

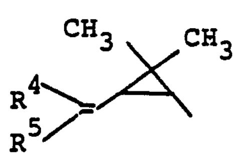 oder 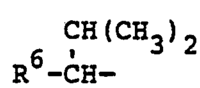

steht,
$R^4$ und $R^5$ für Chlor oder Brom stehen und
$R^6$ für gegebenenfalls substituierter Phenyl steht.

2. Die neuen Benzylester der Formel I werden erhalten, indem man
a) Carbonylhalogenide der Formel II

$$Hal—CO—R^3 \qquad (II)$$

in welcher
$R^3$ die unter 1 (oben) angegebene Bedeutung hat, und
Hal für Halogen, vorzugsweise Chlor, steht, mit Benzylalkoholen der Formel III

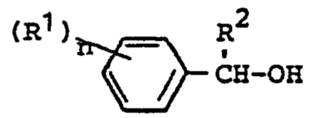

(III)

in welcher

$R^1$, n und $R^2$ die unter 1 (oben) angegebene Bedeutung haben,

gegebenenfalls in Gegenwart eines Säureakzeptors und gegebenenfalls in Gegenwart eines Lösungsmittels umsetzt, oder

b) Carbonsäuresalze der Formel IV

$$MeO—CO—R^3 \quad (IV)$$

in welcher

Me K oder Na bedeutet und

$R^3$ die unter 1 (oben) angegebene Bedeutung hat,

mit Benzylhalogeniden der Formel V

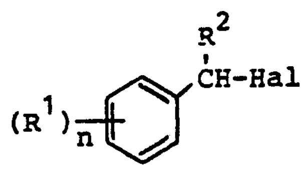

(V)

in welcher

$R^1$, n und $R^2$ die unter 1 (oben) angegebene Bedeutung haben und

Hal Chlor oder Brom bedeutet,

gegebenenfalls in Gegenwart eines Lösungsmittels und gegebenenfalls in Gegenwart eines quartären Ammoniumsalzes umsetzt.

3. Die neuen Benzylester der Formel I und ihre Herstellung sind Gegenstand der Anmeldung EP-A 79 101 242.0. Die neuen Benzylalkohole der Formel III

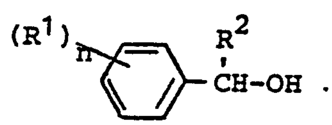

(III)

in welcher

$R^1$, $R^2$ und n die unter 1 (oben) angegebene Bedeutung haben,

werden erhalten, indem man

a) Aldehyde der Formel VI

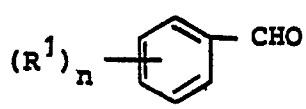

(VI)

in welcher

$R^1$ und n die unter 1 (oben) angegebene Bedeutung haben,

für den Fall, daß $R^2$ im Benzylalkohol für Wasserstoff steht reduziert, oder für den Fall, daß $R^2$ im Benzylalkohol für CN steht mit HCN umsetzt, oder für den Fall, daß $R^2$ im Benzyalkohol für $C_{1-4}$-Alkyl oder Äthinyl steht mit einer Grignard-Verbindung der Formel VII

$$R^2—Mg—Hal \quad (VII)$$

in welcher

$R^2$ für $C_{1-4}$-Alkyl oder Äthinyl steht

umsetzt, oder

b) indem man Benzylamine der Formel VIII

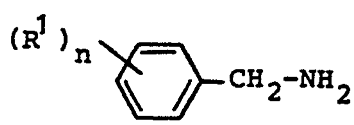

(VIII)

in welcher
$R^1$ und n die unter 1 (oben) angegebene Bedeutung haben,
mit Natrium- oder Kaliumnitrit in Gegenwart einer Säure umsetzt oder
e) indem man ein Benzylhalogenid der Formel V

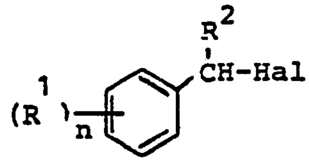

(V)

in welcher
$R^1$, $R^2$, n und Hal die unter 2 (oben) angegebene Bedeutung haben,
mit wäßrigen Basen verseift.

4. Die neuen Benzylhalogenide der Formel V

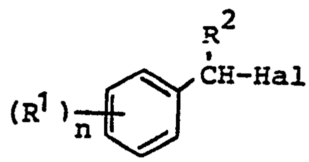

(V)

in welcher
$R^2$ und n die unter 4 (oben) angegebene Bedeutung haben und
$R^1$ für gleiche oder verschiedene Reste aus der Gruppe Wasserstoff, $C_{1-6}$-Alkyl, $C_{1-6}$-Halogenalkyl mit 3-6 Halogenatomen, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, Halogen, gegebenenfalls substituiertes Phenyl, gegebenenfalls substituiertes Phenoxy, steht oder zwei benachbarte Reste $R^1$ gemeinsam mit den angrenzenden C-Atomen einen gegebenenfalls substituierten ankondensierten Benzolring oder ein oder mehrfach durch Fluor substituierte Sauerstoff enthaltende heterocyclische Fünf- oder Sechsringe bilden. Dabei müssen zwei benachbarte Reste $R^1$ gemeinsam mit den angrenzenden C-Atomen einen ein oder mehrfach durch Fluor substituierten Sauerstoff enthaltenden heterocyclischen Fünf- oder Sechsring bilden, werden erhalten, indem man Verbindungen der Formel IX

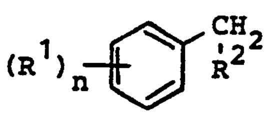

(IX)

in welcher
$R^1$, $R^2$ und n die oben angegebene Bedeutung haben,
in der Seitenkette auf an sich bekannte Weise halogeniert.

5. Die neuen Aldehyde der Formel VI

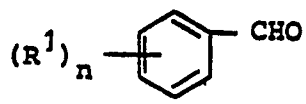

(VI)

in welcher $R^1$ und n die unter 5 (oben) angegebene Bedeutung haben, jedoch mit der Maßgabe, daß zwei benachbarte Reste $R^1$ gemeinsam mit den angrenzenden C-Atomen einen ein oder mehrfach durch Fluor substituierten Sauerstoff enthaltenden Sechsring bilden müssen, werden erhalten, indem man in Verbindungen der Formel XI

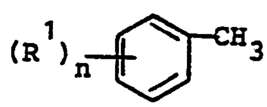

(XI)

in welcher
$R^1$ und n die oben angegebene bedeutung haben, die $CH_3$-Gruppe in an sich bekannter Weise zur —CH—$Hal_2$-Gruppe halogeniert und anschließend in üblicher Weise zum Aldehyd der Formel VI verseift.

Die Verbindungen der Formel I zeigen gute insektizide Eigenschaften.

Von den erfindungsgemäßen Verbindungen der Formel I gemäß 1 (oben) sind bevorzugt diejenigen, in denen

n für 1-5 steht, und

$R^1$ für gleiche oder verschiedene Reste aus der Gruppe Wasserstoff, Fluor, Chlor, Brom, Trifluormethyl, Methoxy, Difluormethoxy, Trifluormethoxy, Tetrafluorethoxy, Hexafluorpropoxy, Difluormethylthio, Trifluormethylthio oder für Phenyl oder Phenoxy, die gegebenenfalls durch Halogen oder Alkoxy substituiert sind, stehen oder zwei benachbarte Reste gemeinsam mit den angrenzenden C-Atomen einen mehrfach durch Fluor substituierten Sauerstoff enthaltenden Fünf- oder Sechsring bilden, wobei zwei der Reste gemeinsam mit den angrenzenden C-Atomen einen heterocyclischen Fünf- oder Sechsring bilden und für $OCF_2O$, $OCF_2CH_2O$ oder $OCF_2CHFO$ stehen,

$R^2$ für Wasserstoff, $C_1$-$C_4$-Alkyl-, Cyan oder Äthinyl steht und

$R^3$ für den Rest

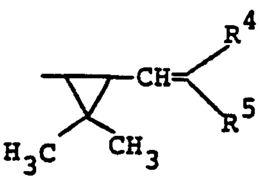

steht, wobei

$R^4$ und $R^5$ gleich oder verschieden sind und für Fluor, Chlor oder Brom oder beide für Methyl stehen, oder $R^3$ für den Rest

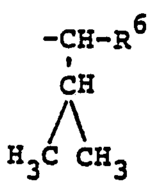

steht, wobei

$R^6$ für einen gegebenenfalls durch Halogen, Alkyl, Alkylthio, Alkoxy mit jeweils 1-4 C-Atomen, Nitro, Methylendioxy substituierten Phenylring steht.

Ganz besonders bevorzugt sind solche Verbindungen der Formel I in welcher $R^1$ für gleiche oder verschiedene Reste der Gruppe Wasserstoff, Chlor, Difluormethoxy, Tetrafluoräthoxy, Hexafluorpropoxy oder Trifluormethylthio steht oder zwei benachbarte Reste $R^1$ gemeinsam mit den angrenzenden C-Atomen einen heterocyclischen Sauerstoff enthaltenden 5- oder 6-Ring bilden, wobei zwei benachbarte Reste gemeinsam mit den angrenzenden C-Atomen einen heterocyclischen 5- oder 6-Ring bilden und für $OCF_2O$, $OCF_2$—$CH_2O$ oder $OCF_2CHFO$ stehen, $R^2$ für Wasserstoff oder Cyan steht und $R^3$ für den Rest

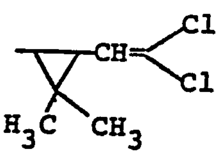

oder für den Rest

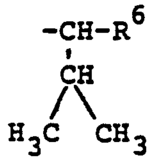

wobei $R^6$ für einen durch Fluor, Chlor oder Brom, Methoxy oder Methylendioxy substituierten Phenylring steht.

Folgende Verbindungen der Formel I seien im einzelnen genannt :

Difluor-3,4-dioxymethylen-benzyl-2,2-dimethyl-3-(2',2'-dichlorvinyl)-cyclopropancarboxylat,
Difluor-2,3-dioxymethylen-benzyl-2,2-dimethyl-3-(2',2'-dichlorvinyl)-cyclopropancarboxylat,
Difluor-3,4-dioxymethylen-6-chlorbenzyl-2,2-dimethyl-3-(2',2'-dichlorvinyl)-cyclopropancarboxylat,
Difluor-3,4-dioxymethylen-6-brombenzyl-2,2-dimethyl-3-(2',2'-dichlorvinyl)-cyclopropancarboxylat,

Difluor-3,4-dioxymethylen-2,5,6-trichlorbenzyl-2,2-dimethyl-3-(2',2'-dichlorvinyl)-cyclopropancarboxylat,
Difluor-3,4-dioxyäthylen-benzyl-2,2-dimethyl-3-(2',2'-dichlorvinyl)-cyclopropancarboxylat,
Trifluor-3,4-dioxyäthylen-benzyl-2,2-dimethyl-3-(2',2'-dichlorvinyl)-cyclopropancarboxylat,
Difluor-3,4-dioxyäthylen-6-chlorbenzyl-2,2-dimethyl-3-(2',2'-dichlorvinyl)-cyclopropancarboxylat,
Trifluor-3,4-dioxyäthylen-6-chlorbenzyl-2,2-dimethyl-3-(2',2'-dichlorvinyl)-cyclopropancarboxylat.

Difluor-3,4-dioxymethylen-benzyl-4'-chlorphenyl-α-isopropylacetat,
Difluor-2,3-dioxymethylen-benzyl-4'-chlorphenyl-α-isopropylacetat,
Difluor-3,4-dioxymethylen-6-chlorbenzyl-4'-chlorphenyl-α-isopropylacetat,
Difluor-3,4-dioxymethylen-6-brombenzyl-4'-chlorphenyl-α-isopropylacetat,
Difluor-3,4-dioxymethylen-2,5,6-trichlorbenzyl-4'-chlorphenyl-α-isopropylacetat,
Difluor-3,4-dioxyäthylen-benzyl-4'-chlorphenyl-α-isopropylacetat,
Trifluor-3,4-dioxyäthylen-benzyl-4'-chlorphenyl-α-isopropylacetat,
Difluor-3,4-dioxyäthylen-6-chlorbenzyl-4'-chlorphenyl-α-isopropylacetat,
Trifluor-3,4-dioxyäthylen-6-chlorbenzyl-4'-chlorphenyl-α-isopropylacetat.

Die Herstellung der erfindungsgemäßen Benzylester der Formel I kann durch folgendes Reaktionsschema wiedergegeben werden :

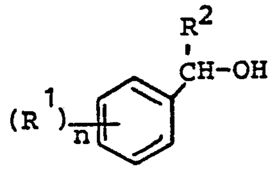
+ Hal-CO-$R^3$ Säureakzeptor
-HCl
(I)

Verwendet man beispielsweise bei der Verfahrensvariante a) gemäß 2 (oben) 2,2-Dimethyl-3-(2',2'-dichlorvinyl)-cyclopropancarbonsäurechlorid und Difluor-3,4-methylendioxybenzylalkohol als Ausgangsmaterialien, so kann der Reaktionsverlauf durch das folgende Formelschema wiedergegeben werden :

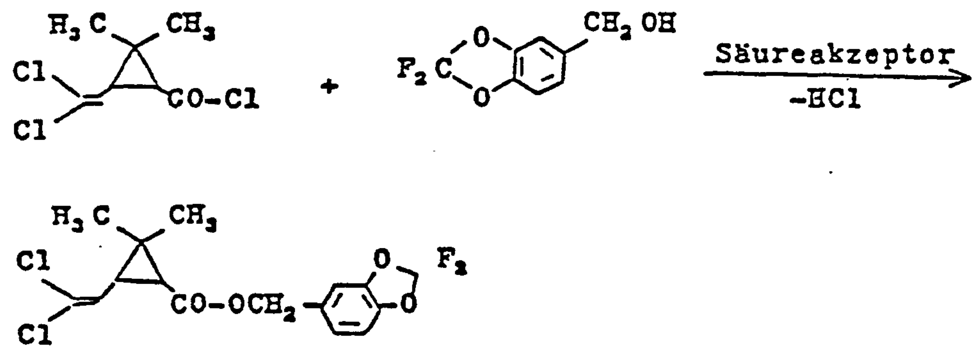


Die als Ausgengsprodukte zur verwendenden Carbonylhalogenide der Formel II sind bekannt und nach allgemein üblichen, in der Literatur beschriebenen Verfahren herstellbar (vgl. z. B. DT-OS 2 365 555 ; 1 926 433 und 2 231 312).

Die ebenfalls als Ausgangsprodukte zu verwendenden Alkohole der Formel III sind neu.

Die neuen Alkohole können nach den unter 3 (oben) angegebenen Verfahren hergestellt werden (Einzelheiten siehe weiter unten).

Bevorzugt werden Alkohole der Formel III verwendet in welcher $R^1$, $R^2$ und n die weiter oben angegebene bevorzugte oder besonders bevorzugte Bedeutung haben.

Als Beispiele für die als Ausgangsprodukte zu verwendenden Alkohole der Formel II seien im einzelnen genannt :

Difluor-3,4-dioxymethylen-benzylalkohol,
Difluor-3,4-dioxymethylen-α-cyano-benzylalkohol,
Difluor-3,4-dioxymethylen-α-äthinyl-benzylalkohol.

Zur Herstellung der erfindungsgemäßen Verbindungen der Formel I gemäß 1 (oben) aus Alkoholen der Formel III und Carbonylhalogeniden der Formel II können als Säureakzeptoren alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 100 °C, vorzugsweise bei 15 bis 40 °C.

Die Umsetzung läßt man im allgemeinen bei Normaldruck ablaufen. Das Verfahren zur Herstellung der erfindungsgemäßen Verbindungen wird bevorzugt unter Mitverwendung geeigneter Lösung- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol oder Äther, z. B. Diäthyl- und Dibutyläther, Dioxan, ferner Ketone, beispielsweise Aceton, Methyläthyl-, Methylisopropyl- und Methylisobutylketon, außerdem Nitrile, wie Aceto- und Propionitril.

Zur Durchführung des Verfahrens setzt man die Ausgangskomponenten vorzugsweise in äquimolaren Verhältnissen ein. Ein Überschuß der einen oder anderen Komponente bringt keine wesentlichen Vorteile. Die Reaktionskomponenten werden im allgemeinen in einem der angegebenen Lösungsmittel zusammen gegeben und meist bei erhöhter Temperatur zur Vervollständigung der Reaktion eine oder mehrere Stunden gerührt. Anschließend gießt man das Reaktionsgemisch in Wasser, trennt die organische Phase ab und wäscht diese mit Wasser nach. Nach dem Trocknen wird das Lösungsmittel im Vakuum abdestilliert.

Die neuen Verbindungen fallen in Form von Ölen an, die sich um Teil nicht unzersetzt destillieren lassen, jedoch durch sogenanntes « Andestillieren » d. h. durch längeres Erhitzen unter vermindertem Druck auf mäßig erhähte Temperaturen von den letzten flüchtigen Anteilen befreit und auf diese Weise gereinigt werden. Zu ihrer Chrakterisierung dient der Brechungsindex.

Ferner sind die erfindungsgemäßen Verbindungen der Formel I durch Umsetzung der Carbonsäuresalze IV mit Benzylhalogeniden der Formel V erhältlich :

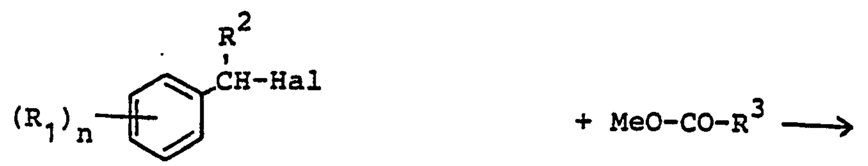

(I)

Verwendet man beispielsweise bei der Verfahrensvariante b) gemäß 2 (oben) das Kaliumsalz der 4-Chlorphenylessigsäure (das gegebenenfalls « in situ » aus Säure und KOH hergestellt werden kann) und 3-Difluormethoxy-benzylbromid als Ausgangsstoffe, so kann der Reaktionsverlauf durch das folgende Formelschema wiedergegeben werden :

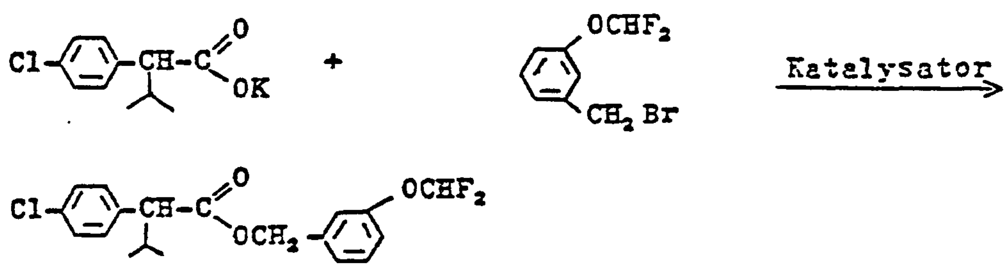


Die als Ausgangsprodukte zu verwendenden Carbonsäuren bzw. ihre Salze der Formel IV sind bekannt und liegen den Carbonylhalogeniden der Formel II zugrunde. Alle den Säurehalogeniden der Formel II zugrundeliegenden Säuren und Salze kommen somit für die hiergenannte Herstellungsweise in Betracht.

Bevorzugt werden Säuren und Salze der Formel IV verwendet in welcher der Rest $R^2$ die weiter oben angegebene bevorzugte oder besonders bevorzugte Bedeutung hat.

Im einzelnen seien die Säuren sowie deren Natrium, Kalium, Calcium oder Ammoniumsalze genannt, die den weiter oben aufgezälten Säurehalogeniden zugrundeliegen.

Die als Ausgangsprodukte verwendeten Benzylhalogenide der Formel V sind zum Teil bekannt. Die neuen Verbindungen können nach weiter unten angegebenen Verfahren erhalten werden. Bevorzugt werden Verbindungen der Formel V verwendet, in der $R^1$ und n die weiter oben angegebene bevorzugte oder besonders bevorzugte Bedeutung hat.

Folgende Benzylhalogenide seien im einzelnen genannt :

Difluor-3,4-dioxymethylenbenzylchlorid,
Difluor-3,4-dioxymethylenbenzylbromid,
Difluor-3,4-dioxymethylen-6-chlorbenzylbromid,
Difluor-3,4-dioxymethylen-6-brombenzylbromid,
Difluor-3,4-dioxymethylen-α-cyanobenzylbromid,

Difluor-2,3-dioxymethylen-benzylchlorid,
Difluor-2,3-dioxymethylen-benzylbromid,
Difluor-3,4-dioxymethylen-2,5,6-trichlorbenzylchlorid.

Zur Herstellung der erfindungsgemäßen Derivate der Formel I aus den Carbonsäuresalzen der Formel IV und den Benzylhalogeniden der Formel V wird üblicherweise ein Lösungsmittel verwendet, wie beispielsweise Benzol, Toluol, Xylol, Chlorbenzol, Dichlorbenzol.

Die Salze der Carbonsäuren können direkt eingesetzt werden oder aber durch Zusatz von KOH oder NaOH in Form ihrer wäßrigen Lösungen, oder in pulverisierter Form « in situ » hergestellt werden. Als Katalysatoren können quartäre Ammoniumsalze, wie beispielsweise Tetrabutylammoniumchlorid, Tetrabutylammoniumbromid, Benzyltriäthylammoniumchlorid oder Methyltrioctyl ammoniumchlorid verwendet werden.

Die Reaktionstemperatur kann innerhalb eines größeren Bereichs variiert werden. Im allgemeinen arbeitet man zwischen 50 und 150 °C, vorzugsweise bei 80 bis 120 °C.

Zur Durchführung des Verfahrens setzt man die Ausgangskomponenten vorzugsweise in äquimolaren Verhältnissen ein. Ein Überschuß des Säuresalzes kann verwendet werden, um eine vollständige Umsetzung des Benzylhalogenids zu erreichen. Die überschüssige Säure kann aus der Wasserphase wiedergewonnen werden. Die Reaktion ist meist nach 1-5 Stunden beendet. Nach Abkühlen des Reaktionsgemisches wird mit Wasser versetzt, die organische Phase abgetrennt und neutral gewaschen. Anschließend wird das Lösungsmittel im Vakuum abdestilliert und die Verbindungen der Formel I wie oben beschrieben gereinigt.

Wie bereits erwähnt sind die Alkohole der Formel III neu. Sie lassen sich nach den unter 3 (oben) angegebenen Verfahren herstellen.

Bei Variante 3a) wird für den Fall, daß in der gewünschten Verbindung der Formell III $R^2$ für Wasserstoff steht der entsprechende Aldehyd mit Wasserstoff reduziert. Dies läßt sich durch folgendes Formelschema darstellen :

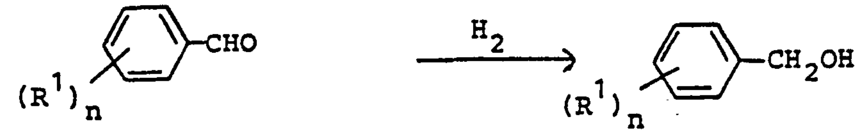


Als Reduktionsmittel kommen Wasserstoff in Anwesenheit von Katalysatoren oder Komplexe Metallhydride wie beispielsweise Natriumborhydrid oder Lithiumaluminiumhydrid in Frage. Die Durchführung der Reaktion erfolgt analog zu bekannten Verfahren (vgl. Organikum, VEB Deutscher Verlag der Wissenschaften, Berlin 1965, 5. Auflage, Seite 417 ; J. Am. Chem. Soc. *71*, 122 (1949) ; *75*, 199 (1953) und *76*, 6116 (1954)).

Für den Fall, daß in der gewünschten Verbindung der Formel III $R^2$ für CN steht wird der entsprechende Aldehyd mit HCN umgesetzt. Dies läßt sich durch folgendes Formelschema darstellen :

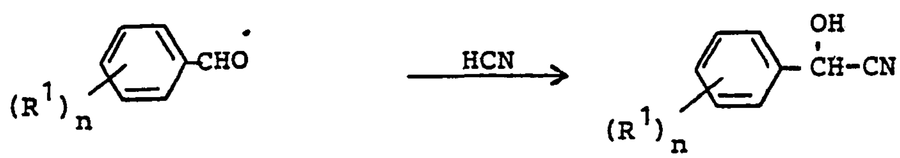


Die Durchführung der Reaktion erfolgt analog den bekannten Verfahren zur Herstellung von Cyanhydrinen (vgl. Organic Syntheses ; Coll. Vol. I, 336 ; Houben-Weyl Band VIII, Seite 274 f.).

Für den Fall, daß in der gewünschten Verbindung der Formel III $R^2$ für $C_{1-4}$-Alkyl oder für Äthinyl steht, wird der entsprechende Aldehyd mit einer Grignard-Verbindung der Formel VII umgesetzt. Dies läßt sich durch folgendes Formelschema darstellen :

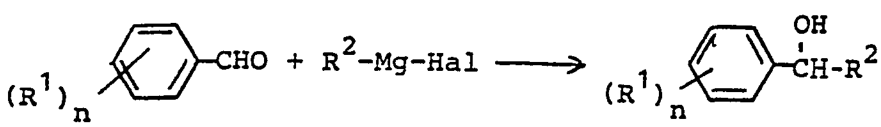

Die Durchführung der Reaktion erfolgt analog den in z. B. in Org. Synth. Coll. Vol. IV, Seite 792 beschriebenen Methoden.

Die Grignard-Verbindungen der Formel VII sind bekannt (vgl. z. B. die oben angegebene Literaturstelle).

Bei Verfahren 3a) werden bevorzugt Aldehyde der Formel VI eingesetzt, in welcher $R^1$ und n die weiter oben angegebene bevorzugte oder besonders bevorzugte Bedeutung besitzen.

Folgende Aldehyde der Formel VI seien im einzelnen genannt :

2-Difluormethoxy-benzaldehyd,
2-Trifluormethylthio-benzaldehyd,
3-Difluormethoxy-benzaldehyd,
3,4-Bis-difluormethoxy-benzaldehyd,
3-Difluormethoxy-4-chlor-benzaldehyd,
3-Trifluormethoxy-benzaldehyd,
3-Trifluormethylthio-benzaldehyd,
Difluor-3,4-dioxymethylen-benzaldehyd.

Die Aldehyde der Formel VI sind zum Teil bekannt (vgl. z. B. J. Org. Chem. *37*, 673 (1972), Z. obsc. Chim. *30*, 3129 (1960)) oder können nach bekannten Verfahren hergestellt werden. Die Difluormethoxyverbindungen erhält man beispielsweise aus den entsprechenden Phenolen mit Difluorchlormethan in Gegenwart von Basen (vgl. z. B. DOS 2 150 955, J. Org. Chem. *25*, 2009 (1960)).

Die neuen Aldehyde der Formel VI können erhalten werden, indem man die Verbindungen der Formel XI gemäß 5 (oben) in an sich bekannter Weise in der Seitenkette zweifach halogeniert, bevorzugt chloriert, und die so erhaltenen Verbindungen in an sich bekannter Weise verseift. Bevorzugt werden dabei Verbindungen der Formel XI eingesetzt, in welcher $R^1$ und n die oben angegebene bevorzugte oder besonders bevorzugte Bedeutung besitzen. Im Einzelnen seien folgende Verbindungen der Formel XI genannt :

Trifluor-3,4-dioxyäthylen-toluol,
Difluor-3,4-dioxyäthylentolyuol,
Trifluor-2,3-dioxyäthylentoluol,
Trifluor-3,4-dioxyäthylen-6-chlor-toluol

Bei Variante 3b) werden Benzylamine der Formel VIII mit salpetriger Säure in Anwesenheit von Säuren, beispielsweise Essigsäure, umgesetzt. Diese Reaktion läßt sich durch folgendes Formelschema darstellen.

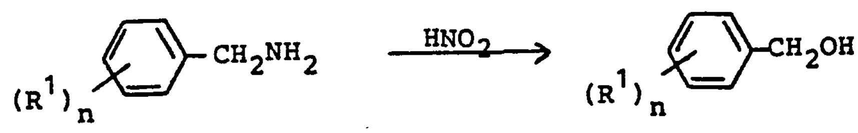


(VIII)

Die dabei verwendeten Benzylamine der Formel VIII sind neu, man erhält sie durch Reduktion der entsprechenden Nitrile mit Wasserstoff analog zu bekannten Verfahren (Houben-Weyl, Band XI/1, Seite 577). Dabei werden die Nitrile z. B. durch Umsetzung der entsprechenden Bromverbindungen mit Kupfer (I) cyanid analog zu bekannten Verfahren erhalten (Houben-Weyl, Band VIII, S. 302). Der Gesamtverlauf der Reaktion läßt sich wenn beispielsweise zwei Reste $R^1$ gemeinsam $OCF_2O$ bedeuten und $R^2$ für Wasserstoff steht durch folgendes Formelschema darstellen :

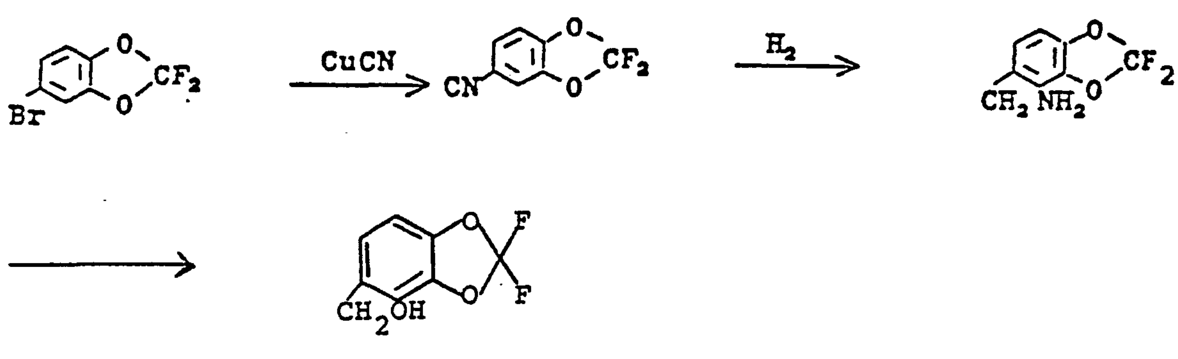


Bei Variante 4c) werden die Benzylhalogenide der Formel V verseift. Diese Reaktion läßt sich durch folgendes Formelschema darstellen :

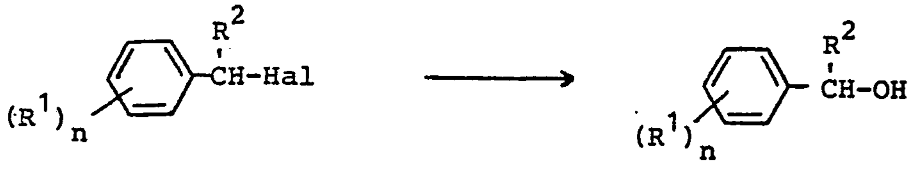

(V)

Die Verseifung erfolgt dabei in an sich bekannter Weise mit wäßrigen Basen, wie beispielsweise NaOH, KOH oder Alkalicarbonate, wie $Na_2CO_3$ oder $K_2CO_3$.

Die bei Verfahren 2b) sowie 3c) (oben) verwendeten Benzylhalogenide der Formel V wind zum Teil neu. Die neuen Verbindungen sind z. B. wie unter 4 (oben) angegeben erhältlich indem man nach im Prinzip bekannten Methoden Verbindungen der Formel IX halogeniert, insbesondere bromiert oder chloriert.

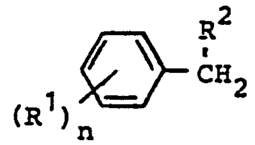

(IX)

Als Halogenierungsmittel kommen beispielsweise Chlor oder N-Chlor- oder N-Bromsuccinimid in Frage.

Die Chlorierung oder Bromierung der vorgenannten Verbindungen zu den ensprechenden Benzylchloriden oder -bromiden erfolgt in an sich bekannter Weise unter radikalischen Bedingungen mit Chlor, N-Chlorsuccinimid oder N-Brom-succinimid in Lösungsmitteln wie beispielsweise Methylenchlorid, Chloroform, Tetrachlorkohlenstoff, Chlorbenzol, o-Difluorbenzol, vorzugsweise bei erhöhter Temperatur.

Bevorzugte Ausgangsverbindungen der Formel IX sind solche in welcher $R^1$ und n für die oben angegebenen bevorzugten oder besonders bevorzugten Definitionen steht. Als einzelne Verbindungen seien genannt :

Difluor-3,4-dioxymethylen-toluol
Difluor-3,4-dioxymethylen-6-chlor-toluol
Difluor-2,3-dioxymethylen-toluol
Trifluor-3,4-dioxyäthylen-toluol
Difluor-3,4-dioxyäthylentoluol
Trifluor-2,3-dioxyäthylentoluol
Difluor-2,3-dioxyäthylentoluol
1-n-Propyl-difluor-3,4-methylendioxy-benzol.

Ein Teil der bei den Verfahren gemäß 4 und 5 oben erwähnten Ausgangsverbindungen ist neu.

Bevorzugt sind Verbindungen der Formel XIV bei denen ein Rest $R^1$ $C_{1-6}$-Alkyl bedeutet und die anderen Reste $R^1$ für gleiche oder verschiedene Reste aus der Gruppe Wasserstoff, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, Halogen, gegebenenfalls substituiertes Phenyl oder zwei benachbarte Reste gemeinsam mit den angrenzenden C-Atomen einen gegebenenfalls substituierten ankondensierten Benzolring bilden, stehen.

Ganz besonders bevorzugt sind dabei die Verbindungen XIV, bei denen ein Rest $R^1$ Methyl bedeutet und die anderen Reste $R^1$ für gleiche oder verschiedene Reste aus der Gruppe Wasserstoff oder Halogen stehen.

Im einzelnen seien genannt :

Difluor-3,4-dioxymethylen-toluol
Difluor-3,4-dioxymethylen-6-fluor-toluol
Difluor-3,4-dioxymethylen-6-chlor-toluol
Difluor-3,4-dioxymethylen-6-brom-toluol
Difluor-3,4-dioxymethylen-2,5,6-trichlortoluol
Difluor-3,4-dioxymethylen-2,5,6-trifluortoluol
Difluor-2,3-dioxymethylen-toluol.

Es ist bekannt, 2,2-Difluorbenzodioxole aus 2,2-Dichlorbenzodioxolen durch Umsetzung mit Antimontrifluorid zu gewinnen (Z. obsc. Khim. 30 (1960) Nr. 9, 3129-3132). Dies ist jedoch kein technisch verwertbares Verfahren, da Antimontrifluorid recht teuer ist und bei der genannten Methode in wäßrige Lösungen von Antimonchloriden überführt wird, die nur aufwendig zurückgewonnen werden können.

Es wurde nun gefunden, daß man Verbindungen der Formel XIV erhält, wenn man Verbindungen der Formel XV

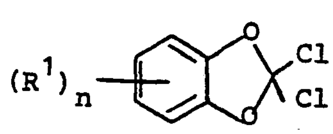

(XV)

in welcher $R^1$ und n die oben angegebene Bedeutung besitzen mit wasserfreier Flußsäure umsetzt.

Die Ausgangstoffe der Formel XV sind teilweise bekannt. Sie können aus den entsprechenden Benzodioxolen (J. Chem. Soc. *93*, 566 (1908)), den entsprechenden Brenzcatechincarbonaten (Chem. Ber. *96*, 1382 (1963)) oder den entsprechenden Brenzkatechinorthoameisensäureestern (Chem. Ber. *94*, 544 (1961)) durch Umsetzung von $PCl_3$ hergestellt werden.

Bevorzugt werden Ausgangsstoffe der Formel XV eingesetzt, in welcher $R^1$ für gleiche oder verschiedene Reste aus der Gruppe Wasserstoff, $C_{1-4}$-Alkyl wie Methyl, Äthyl, n-Tropyl, i-Propyl, t-Butyl, Trichlormethyl, Fluor, Chlor, Brom, Phenyl gegebenenfalls durch Halogen, $C_{1-4}$-Alkyl z. B. Methyl, substituiert, Chlorcarbonyl, Chlorsulfonyl, Nitro oder zwei benachbarte Reste gemeinsam mit den angrenzenden C-Atomen einen ankondensierten Benzolring bilden, stehen.

Als Beispiele für die Ausgangsstoffe der Formel XV seien im einzelnen genannt :

2,2-Dichlorbenzodioxol,
4-Methyl-2,2-dichlorbenzodioxol,
5-Methyl-2,2-dichlorbenzodioxol,
5-Äthyl-2,2-dichlorbenzodioxol,
5-Propyl-2,2-dichlorbenzodioxol,
5-Isopropyl-2,2-dichlorbenzodioxol,
4-Methyl-2,2,5,6-tetrachlorbenzodioxol,
5-Methyl-2,2,6-trichlorbenzodioxol,
5-Methyl-2,2,4,6-tetrachlorbenzodioxol,
5-Propyl-2,2,6-trichlorbenzodioxol,
5-Methyl-6-brom-2,2-dichlorbenzodioxol,
5-Fluor-2,2-dichlorbenzodioxol,
5-Brom-2,2-dichlorbenzodioxol,
2,2,5-Trichlorbenzodioxol,
4-Phenyl-2,2-dichlorbenzodioxol,
5-Phenyl-2,2-dichlorbenzodioxol,
4-Methoxy-2,2-dichlorbenzodioxol,
5-Methoxy-2,2-dichlorbenzodioxol,
4-Phenoxy-2,2-dichlorbenzodioxol,
5-(3'-Methyl)-phenoxy-2,2-dichlorbenzodioxol,
4-(4'-Nitro)-phenoxy-2,2-dichlorbenzodioxol,
5-tert.-Butyl-2,2-dichlorbenzodioxol,
5-Chlor-6-nitro-2,2-dichlorbenzodiozol,
2,2,4,6-Tetrachlorbenzodiozol,
2,2,5,6-Tetrachlorbenzodiozol,
5-Methyl-2,2,4,6,7-pentachloro-benzodiozol,
4-Chlorcarbonyl-2,2-dichlorbenzodioxol,
5-Chlorcarbonyl-2,2-dichlorbenzodioxol,
5-Nitro-2,2-dichlorbenzodioxol,
2,2-Dichlor-naphtho-2,3-dioxol,
2,2-Dichlor-naphtho-1,2-dioxol.

Die Umsetzung kann bei dem erfindungsgemäßen Verfahren bei Temperaturen von – 20 °C bis 80 °C erfolgen, besonders bevorzugt sind Temperaturen von 0 °C bis 40 °C.

Die Flußsäure muß mindestens in stöchiometrischen Mengen eingesetzt werden, doch ist im allgemeinen ein Überschuß günstig. So beträgt die Flußsäuremenge bevorzugt die zwei- bis dreifache stöchiometrische Menge, doch kann der Überschuß größer sein. Die Reaktion kann in Gegenwart von Lösungsmitteln wie auch ohne solche ausgeführt werden. Als Lösungsmittel kommen ganz allgemein inerte, aprotische Flüssigkeiten in Frage. Beispielsweise lassen sich Methylenchlorid, Trichlorfluormethan, Tetrachlorkohlenstoff, Chlorbenzol oder Nitrobenzol erfolgreich verwenden. Die Menge des Lösungsmittels ist für das erfindungsgemäße Verfahren nicht von Bedeutung. So wird die Reaktion vorzugsweise ohne Lösungsmittel durchgeführt, wenn die Ausgangsmaterialien flüssig sind.

Im allgemeinen erfolgt die Umsetzung bei Normaldruck, doch läßt sie sich auch bei Überdruck ausführen.

Das erfindungsgemäße Verfahren kann wie folgt durchgeführt werden :

Im Reaktionsgefäß wird bei ca. – 10 °C die benötigte Menge an wasserfreier Flußsäure vorgelegt und unter Rühren das 2,2-Dichlorbenzodioxol zugetropft. Man wählt die Temperatur dann so, daß die Chlorwasserstoff-Entwicklung sofort einsetzt und leitet das entstehende Gas über einen Rückflußkühler in eine Vorlage, wo es kondensiert oder neutralisiert wird. Nach Ende der Zugabe kann die Temperatur noch etwas erhöht werden. Es wird bis zum Ende der Gasentwicklung gerührt und anschließend die überschüssige Flußsäure unter Normaldruck oder vermindertem Druck abdestilliert. Als Rückstand verbleibt das Reaktionsprodukt das dann beispielsweise durch Destillation gereinigt werden kann.

Gegenüber dem bekannten Verfahren zur Herstellung von 2,2-Difluorbenzodioxolen weist das erfindungsgemäße Verfahren den Vorteil auf, einfach durchführbar zu sein und keine ökologisch

bedenklichen Abwässer zu verursachen. Weiterhin kann das überschüssige Fluorierungsmittel einfach zurückgewonnen und erneut in die Reaktion eingesetzt werden.

Die weiteren bei den Verfahren gemäß 4 und 5 oben erwähnten neuen Ausgangsverbindungen sowie ähnliche Verbindungen können nach dem erfindungsgemäßen Verfahren erhalten werden.

Dabei werden Brenzkatechine der Formel

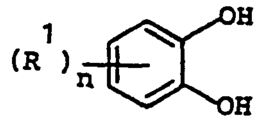

(XVII)

in welcher $R^1$ und n die unter 11b) (oben) angegebene Bedeutung besitzen, in Gegenwart von Basen mit Verbindungen der Formel XVIII

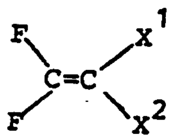

(XVIII)

in welcher

$X^2$ für Halogen, bevorzugt für F, Cl, und

$X^1$ für Wasserstoff oder Halogen, bevorzugt F, Cl, steht,

umgesetzt.

Verwendet man 4-Methylbrenzkatechin und Trifluorchloräthylen als Ausgangsprodukte, läßt sich der Reaktionsablauf durch folgendes Formelschema darstellen :

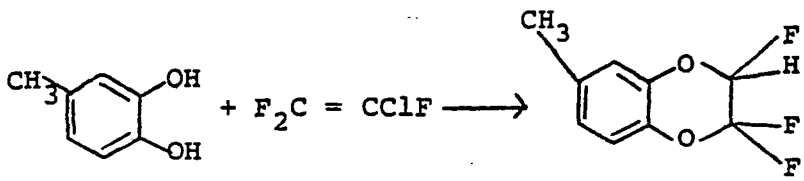

Als Ausgangsprodukte seien bevorzugt Brenzkatechin der Formel XVIII genannt, in welcher $R^1$ für gleiche oder verschiedene Reste aus der Gruppe Wasserstoff $C_{1-4}$-Alkyl insbesondere Methyl, Äthyl, t-Butyl, Fluor, Chlor, Brom, gegebenenfalls durch $C_{1-4}$-Alkyl, insbesondere Methyl, Halogen insbesondere Chlor, Brom, substituiertes Phenyl, Carboxyl (COOH), Nitro, CN, $SO_3H$ steht, oder zwei der Reste gemeinsam mit dem angrenzenden C-Atomen einen ankondensierten Benzolring bilden.

Folgende Verbindungen der Formel XVII seien im einzelnen genannt :

Benzkatechin
3-Methylbrenzkatechin
4-Methylbrenzkatechin
4-tert.-Butyl-brenzkatechin
4-Chlor-brenzkatechin
4-Brom-brenzkatechin
3,5-Dichlor-brenzkatechin
4,5-Dichlorbrenzkatechin
3,4,5-Trichlorbrenzkatechin
Tetrachlorbrenzkatechin
3,4-Dihydroxybiphenyl
2,3-Naphthalindiol
4-Methyl-5-chlor-brenzkatechin
4-Methyl-3,5,6-trichlorbrenzkatechin
2,3-Dihydroxy-benzoesäure
3,4-Dihydroxy-sulfonsäure
4-Nitrobrenzkatechin
4-Cyanobrenzkatechin
2,3-Dihydroxy-terephthalsäure.

Als Beispiele für Verbindungen der Formel XVIII seien im einzelnen genannt :

Chlortrifluoräthylen
Tetrafluoräthylen
Bromtrifluoräthylen

Chlordifluoräthylen
1,1-Dichlor-2,2-difluoräthylen
Bromdifluoräthylen.

Besonders bevorzugt sind Chlortrifluoräthylen und Chlordifluoräthylen.

Als Basen sind besonders die Hydroxide der Alkali- und Erdalkalimetalle geeignet, ebenso aber auch die Carbonate dieser Metalle. Ihre Menge kann zwischen einem und mehr als drei Mol pro Mol Brenzkatechin schwanken. Ein Überschuß wird sich günstig aus. Die Umsetzung kann bei Temperaturen von 20 °C bis 150 °C erfolgen, besonders bevorzugt im Bereich von 80-120 °C. Als Lösungsmittel werden polare Flüssigkeiten verwendet. Es haben sich beispielsweise : Dimethylsulfoxid, Dimethylformamid und Tetramethylensulfon, aber auch Äther wie Dioxan oder Diglyme bewährt. Bevorzugt ist Tetramethylensulfon.

Die Umsetzung erfolgt bei Normaldruck oder bei erhöhtem Druck, um das Entweichen der fluorierten Äthylene zu verhindern, wenn sie beim Zudosieren nicht sofort reagieren. Man arbeitet im allgemeinen zwischen 1-30 bar, bevorzugt zwischen 1-15 bar.

Das erfindungsgemäße Verfahren kann bei 1 bar wie folgt durchgeführt werden :

Im Reaktionsgefäß wird das brenzkatechin im Lösungsmittel gelöst vorgelegt, die Base zugesetzt und unter Rührren auf Reaktionstemperatur erwärmt (beispielsweise 100 °C). Etwa nach einer halben Stunde wird dann in dem Maße das fluorierte Äthylen eingeleitet, wie es von der Reaktionslösung aufgenommen wird. Gegen Ende der Reaktion geht unverbrauchtes Olefin durch, was wieder in die Reaktion zurückgeführt werden kann. Arbeitet man bei Überdruck, werden die Brenzkatechine, Basen und Lösungsmittel in einem Druckgefäß vorgelegt auf die Reaktionstemperatur erwärmt und dann die Äthylene der Formel XVII mit der geschwindigkeit zugepumpt, daß sich der gewünschte Druck einstellt.

Ist die Reaktion beendet, so wird entweder durch fraktionierte Destillation oder durch Verdünnen mit Wasser und Abtrennen des Reaktionsproduktes aufgearbeitet.

Wie bereits erwähnt, zeigen die Verbindungen der Formel I insektizide Wirksamkeit.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophaus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips fermoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypieiia, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp.,

Oryzaephilus surinamensis, Anthonomus spp., Sitophilus spp., Othiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

## Beispiel A

$LT_{100}$-Test für Dipteren

Testtiere : Musca domestica (resistent)
Zahl der Tosttiere : 20
Lösungsmittel : Aceton

2 Gewichtsteile Wirkstoff werden in 1 000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z. B. die folgenden Verbindungen der Herstellungsbeispiele überlegene Wirkung gegenüber dem Stand der Technik : 6, 9, 1.

## Beispiel B

$LT_{100}$-Test für Dipteren

Testtiere : Aedes aegypti
Zahl der Testtiere : 20
Lösungsmittel : Aceton

2 Gewichtsteile Wirkstoff werden in 1 000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z. B. die folgenden Verbindungen der Herstellungsbeispiele überlegene Wirkung gegenüber dem Stand der Technik : 1, 6, 9.

## Beispiel C

Mückenlarven-Test

Testtiere : Aedes aegypti, 4 Larven
Lösungsmittel : Aceton Gewichtsteile 99
Emulgator : Benzylhydroxydiphenylpolyglykoläther Gewichtsteile 1

Zur Herstellung einer zweckmäßigen Wirkstoffzubereitung löst man 2 Gewichtsteile Wirkstoff in 1 000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Gläser und setzt anschließend etwa 25 Mückenlarven in jedes Glas ein.

Nach 24 Stunden wird der Abtötungsgrad in % bestimmt. Dabei bedeutet 100 %, daß alle Larven abgetötet worden sind. 0 % bedeutet, daß überhaupt keine Larven abgetötet worden sind.

Bei diesem Test zeigen z. B. die folgenden Verbindungen der Herstallungsbeispiele überlegene Wirkung gegenüber dem Stand der Technik : 6, 9, 1.

## Beispiel D

Laphygma-Test

Lösungsmittel : 3 Gewichtsteile Dimethylformamid
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das

Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden : 0 % bedeutet, daß keine Raupen abgetötet wurden.

Bei diesem Test zeigen z. B. die folgenden verbindungen der Herstellungsbeispiele überlegene Wirksamkeit gegenüber dem Stand der Technik : 1, 9, 6.

## Herstellungsbeispiele

### Beispiel 1

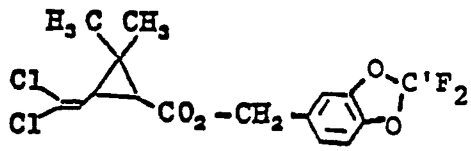

0,035 Mol Difluor-3,4-methylendioxy-benzylalkohol und 3,5 g (0,035 Mol) Triäthylamin werden in 50 ml Toluol gelöst und bei 20-25 °C zu einer Mischung von 7,95 g (0,35 Mol) 2,2-Dimethyl-3-(2',2'-dichlorvinyl)-cyclopropancarbonsäurechlorid in 100 ml Toluol getropft. Man rührt 3 h bei Raumtemperatur nach, gießt das Reaktionsgemisch in 150 ml Wasser, trennt die Toluolphase ab und wäscht sie mit 100 ml Wasser ; trocknet mit Natriumsulfat und destilliert das Toluol i. V. ab. Letzte Lösungsmittelreste werden durch Andestillieren bei einer Badtemperatur von 60 °C/0,2 bis 1,0 Torr entfernt. Man erhält Difluor-3,4-methylendioxy-benzyl-2,2-dimethyl-3-(2',2'-dichlorvinyl)-cyclopropancarboxylat als viskoses Öl mit dem Brechungsindex $n_D^{20}$ = 1,507 0.

Analog Beispiel 1 werden erhalten :

| Beispiel | Formel | Brechungsindex $n_D^{20}$ |
|---|---|---|
| 2 | 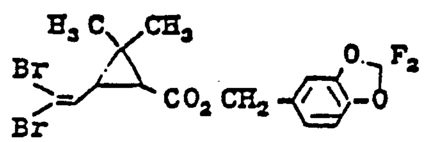 | 1.5230 |
| 3 | 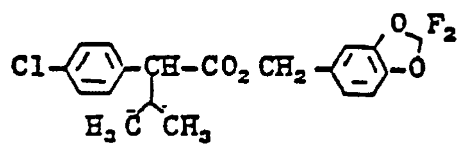 | 1,5170 |
| 4 | 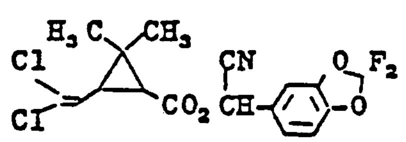 | 1.5062 |

### Beispiel 5

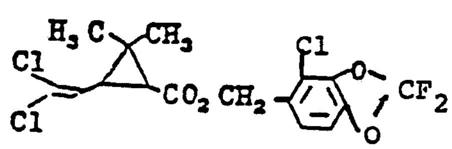

0,03 Mol 2-Chlor-difluor-3,4-methylendioxy-benzylbromid und 6,27 g (0,03 Mol) 2,2-Dimethyl-3-(2',2'-dichlorvinyl)-cyclopropancarbonsäure werden in 150 ml Toluol gelöst und anschließend 0,5 g Tetrabutylammoniumbromid und 2 g pulverisierte KOH (technisch 87 %ig) zugegeben und 2-3 h zum Sieden erhitzt. Nach dem Abkühlen gießt man das Reaktionsgemisch in 150 ml Wasser, trennt die Toluolphase ab und

wäscht sie mit 100 ml Wasser; trocknet mit Natriumsulfat und destilliert das Toluol i.V. ab. Letzte Lösungsmittelreste werden durch Andestillieren bei einer Badtemperatur von 60-80 °C/0,2 bis 1 Torr entfernt. Man erhält 2-Chlor-difluor-3,4-methylendioxybenzyl-2,2-dimethyl-3-(2',2'-dichlorvinyl)-cyclopropancarboxylat als viskoses Öl mit dem Brechungsindex $n_D^{20}$ = 1.511 0.

Analog Beispiel 5 werden erhalten:

| Beispiel | Formel | Brechungsindex $n_D^{20}$ |
|---|---|---|
| 6 | 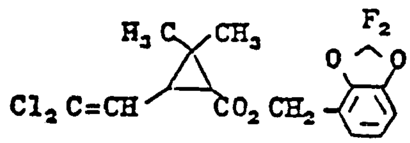 | 1.5080 |
| 7 | 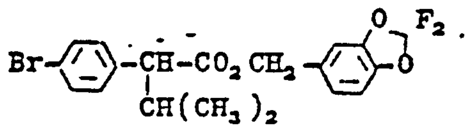 | 1.5205 |
| 8 | 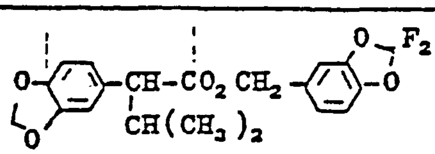 | 1.4992 |
| 9 | 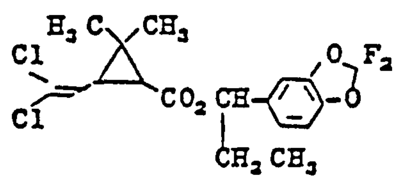 | 1.5164 |
| 10 | 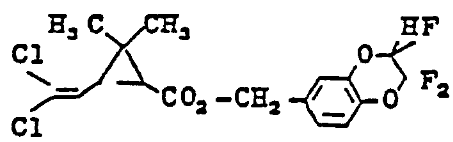 | 1.5090 |
| 11 | 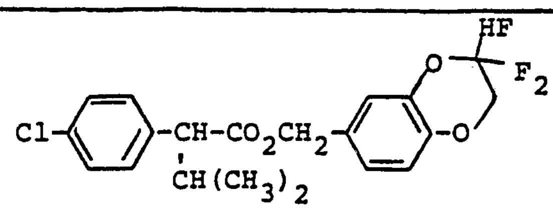 | 1.5149 |

Herstellung der Ausgangsstoffe

1. 2-Difluormethoxybenzylalkohol

Man löst 51,5 g (0,3 Mol) 2-Difluormethoxybenzaldehyd in 100 ml Äthanol und tropft 6,25 g Natriumborhydrid in 100 ml Äthanol bei 25-35 °C zu. Anschließend rührt man nach bis Raumtemperatur erreicht ist, verdünnt mit Wasser und säuert mit 25 %iger $H_2SO_4$ an. Nach zweimaligem Extrahieren mit Methylenchlorid wird die organische Phase getrocknet und das Lösungsmittel abdestilliert. Man erhält

ein farbloses Öl in nahezu quantitativer Ausbeute, das nach einiger Zeit erstarrt. Der 2-Difluormethoxybenzylalkohol schmilzt bei 42 °C.

2. 2- und 3-Trifluormethylthiobenzylalkohol

Werden analog dem vorstehenden Beispiel aus 2-Trifluormethylthiobenzaldehyd erhalten.

3. 2-Difluormethoxy-α-cyano-benzylalkohol

7,2 g Kaliumcyanid werden unter Kühlung in 30 ml Wasser und 6 ml Äthanol gelöst. Dann wird unter Kühlung bei 0-5 °C 16 g 2-Difluormethoxybenzaldehyd zugegeben. Nach 20 Min. Nachrühren wird zwischen 0 und 10 °C eine Mischung aus 7 ml konz. $H_2SO_4$ und 18 ml Wasser zugetropft. Man rührt noch 2 h nach und läßt dabei auf Raumtemperatur kommen. Nach zweimaligem Extrahieren mit Methylenchlorid wird die organische Phase getrocknet und das Lösungsmittel abdestilliert. Man erhält 2-Difluormethoxy-α-cyano-benzalkohol als farbloses Öl, dessen Struktur durch ein Kernresonanzspektrum bestätigt wird. Die Ausbeute ist praktisch quantitativ.

4. Analog vorstehendem Beispiel erhält man 2-Trifluormethylthio-α-cyano-benzylalkohol aus 2-Trifluormethylthio-benzaldehyd und Difluor-3,4-dioxymethylen-α-cyano-benzylalkohol aus Difluor-3,4-dioxymethylen-benzaldehyd.

5. Analog Beispiel 1, erhält man aus Difluor-3,4-dioxymethylenbenzaldehyd durch Reduktion mit Natriumborhydrid Difluor-3,4-dioxymethylen-benzylalkohol. Das NMR-Spektrum (in $CDCl_3$) bestätigt die Struktur : 3,7 ppm (s, OH) ; 4,6 ppm (s, $CH_2$) ; 7,0 ppm und 7,05 ppm (3. aromat. H).

6. Difluor-3,4-methylendioxy-toluol

Im Reaktionsgefäß werden bei 0 °C 200 ml HF (wasserfrei) vorgelegt und 190 g Dichlor-3,4-methylendioxytoluol (vgl. J. Chem. Soc. 93, 563) zugetropft. Nach Ende der Chlorwasserstoffentwicklung wird auf 20 °C erwärmt, 1 h nachgeführt und anschließend die überschüssige Flußsäure bei vermindertem Druck abdestilliert. Difluor-3,4-methylendioxy-toluol siedet bei 74-78 °C/52 mm ($n_D^{20}$ = 1.492).

7. 1-n-Propyl-difluor-3,4-methylendioxy-benzol erhält man analog der vorstehenden Vorschrift. Kp = 80°/15 mm ($n_D^{20}$ = 1.454 0).

8. Difluor-3,4-methylendioxy-benzylbromid

172 g (1 Mol) Difluor-3,4-methylendioxytoluol, 180 g N-Bromsuccinimid und eine Spatelspitze Azobisisobutyronitril werden mit 1 000 ml $CCl_4$ gemischt und 5 h zum Sieden erhitzt. Nach dem Abkühlen wird abfiltriert, mit etwas $CCl_4$ nachgewaschen und das Filtrat destilliert. Man erhält 180 g (72 % d. Th.) Difluor-3,4-methylendioxy-benzylbromid vom Kp = 180-111 °C/15 mm. Brechungsindex $n_D^{20}$ = 1.518.

9. Analog vorstehendem Beispiel erhält man 1-Brom-1-(difluor-3,4-methylendioxy)-phenyl-propan vom Kp = 70-73 °C/0,3 mm.

10. Difluor-3,4-methylendioxy-6-chlor-benzylbromid

In eine Lösung von 34,4 g (0,2 Mol) Difluor-3,4-methylendioxy-toluol in 40 ml Methylenchlorid werden bei − 10 °C bis − 5 °C 14 g Chlor eingeleitet. Man läßt auf Raumtemperatur kommen und fraktioniert im Wasserstrahlvakuum. Man erhält 31 g Difluor-3,4-methylen-dioxy-6-chlor-toluol vom Kp = 80-84°/15 mm, die in 150 ml Tetrachlorkohlenstoff gelöst werden. Nach Zugabe von 33 g N-Brom-succinimid und einer Spatelspitze Azibisisobutyronitril erhitzt man 5 h zum Sieden. Man erhält 30 g Difluor-3,4-methylendioxy-6-chlor-benzylbromid vom Kp = 74-75 °C/0,25 mm Brechungsindex $n_D^{20}$ = 1.533 4.

11. Trifluor-3,4-dioxyäthylen-benzylbromid

Man löst 124 g 4-Methylbenzkatechin in 300 ml Tetramethylensulfon und fügt 110 g KOH hinzu. Anschließend leitet man bei 100° bis 110 °C 170 g Trifluorchloräthylen ein. Nach dem Abkühlen destilliert man über eine Kolonne im Wasserstrahlvakuum. Man erhält 132 g Trifluor-3,4-dioxyäthylen-toluol vom Kp 70-72 °C/12 mm $n_D^{20}$ = 1.456 5.

50 g werden mit 50 g N-Bromsuccinimid in 150 ml $CCl_4$ analog vorstehender Vorschrift bromiert (8 h Rückfluß). Das Trifluor-3,4-dioxyäthylen-benzylbromid siedet bei 123-124 °C/13 mm ; Brechungsindex $n_D^{20}$ = 1.516 5.

12. 4-Methyl-2,2-difluorbenzodioxol

Im Reaktionsgefäß werden bei 0 °C 200 ml HF vorgelegt und 100 g 4-Methyl-2,2-dichlorbenzodioxol zugetropft. Nach Ende der Chlorwasserstoffentwicklung wird noch auf 20 °C erwärmt, 1 Stunde nachgerührt und anschließend die überschüssige Flußsäure bei vermindertem Druck abdestilliert. Das 4-Methyl-2,2-difluorbenzodioxol hat einen Siedepunkt von 42-45 °C bei 11 mm ($n_D^{20}$ : 1.451 5).

Das 4-Brommethyl-2,2-difluorbenzodioxol siedet bei 93-96 °C/10 mm ($n_D^{20}$ = 1.511 5).

13. 5-Propyl-2,2-difluorbenzodioxol

Bei – 2 °C werden 100 ml wasserfreie Flußsäure im Reaktionsgefäß vorgelegt und anschließend 110 g 5-Propyl-2,2-dichlorbenzodioxol zugetropft. Nach analoger Durchführung wie im Beispiel 15 a erhält man 67 g 5-Propyl 2,2-difluorbenzodioxol mit einem Siedepunkt von 80-83 °C bei 15 mm ($n_D^{20}$ : 1.454 0).

14. Difluormethylen-3,4-dioxybenzoesäure

100 ml wasserfreie Flußsäure werden bei – 3 °C vorgelegt und dann 55 g 5-Chlorcarbonyl-2,2-dichlorbenzodioxol zugetropft. Nach Ende der Zugabe wird noch auf 20 °C erwärmt und bis zum Ende der Chlorwasserstoffentwicklung gerührt. Dann wird die überschüssige Flußsäure abdestilliert und der Rückstand in 200 ml 5 %ige Natronlauge eingerührt. Die Lösung wird filtriert und anschließend mit Salzsäure angesäuert. Die ausgefallene Difluormethylen-3,4-dioxybenzoesäure wird abgesaugt und getrocknet. Man erhält 35 g Säure mit einem Schmelzpunkt von 153-154 °C.

15. 2,2-Difluorbenzodioxol

a) In einem Reaktionsgefäß aus $V_4A$ mit Rührer, Rückflußkühler und Tropftrichter werden bei – 10 °C 600 g wasserfreie Flußsäure vorgelegt. Dann werden 612 g 2,2-Dichlorbenzodioxol unter Feuchtigkeitsausschluß in ca. 2 h zugetropft. Es setzt sofort Chlorwasserstoffentwicklung ein. Das Gas wird durch eine Ableitung vom Rückflußkühler in eine Vorlage mit Wasser geleitet und absorbiert. Nach Ende der Zugabe wird die Temperatur auf 18°-20 °C erhöht und noch 1 Stunde bis Ende der Gasentwicklung nachgerührt. Die überschüssige Flußsäure wird nun über eine Kolonne abdestilliert und in einer gekühlten Vorlage aufgefangen. Anschließend destilliert man bei 100 mm Druck mit einem Siedepunkt von 65-70 °C 394 g 2,2-Difluorbenzodioxol über ($n_D^{20}$ : 1.443 0). Die Ausbeute beträgt 78 % d. Th.

b) Zu 200 ml wasserfreier Flußsäure im Reaktionsgefäß aus $V_4A$ tropft man unter Rühren eine Lösung von 150 g 2,2-Dichlorbenzodioxol in 200 ml Methylenchlorid. Die Reaktion setzt bei 0 °C sofort ein. Man läßt bei 0 °C ausreagieren, erhöht dann die Temperatur auf 20 °C und rührt für eine weitere Stunde. Dann destilliert man die überschüssige Flußsäure und das Lösungsmittel bei vermindertem Druck ab und destilliert anschließend das 2,2-Difluorbenzodioxol über. Man erhält 85 g Produkt, das entspricht 68 % d. Th.

16. 2,2-Difluor-5-chlor-benzodioxol wird analog Beispiel 15a erhalten. Kp = 57 °C/14 mm ($n_D^{20}$ = 1.471 2).

17. 2,2-Difluor-1,4-benzodioxen

110 g Brenzkatechin werden zusammen mit 70 g Kaliumhydroxid in 300 ml Tetramethylensulfon vorgelegt und unter Rühren innerhalb von 30 Min. auf 100 °C erhitzt. Bei einer Temperatur von 100-110 °C werden 140 g 1,1-Difluor-2-chloräthylen eingeleitet (Dauer ca. 3 Stunden). Anschließend wird bei 15 mm das Produkt über eine kleine Kolonne in eine gute gekühlte Vorlage destilliert. Man erhitzt hierbei bis zu einer Innentemperatur von 100 °C. Der Inhalt der Vorlage wird in einem Scheidetrichter überführt und die organische Phase von der wäßrigen abgetrennt. Man erhält 112 g ≙ 65 % d. Th. an 2,2-Difluor-1,4-benzodioxen mit einem Brechungsindex $n_D^{20}$ : 1.480 2, das nach GC-Analyse rein ist.

18. 6-Methyl-2,2,3-trifluor-1,4-benzodioxen

124 g 4-Methylbrenzkatechin werden zusammen mit 110 g Kaliumhydroxid in 300 ml Tetramethylensulfon bei 110 °C vorgelegt. Es werden dann innerhalb 4 Stunden 170 g Trifluorchloräthylen eingeleitet. Der Ansatz wird anschließend bei 15 mm über eine Kolonne destilliert, wobei bis zu einer Übergangstemperatur von 85 °C abgenommen wurde. Nach Abtrennen der wäßrigen Phase in der Vorlage wird das Produkt erneut destilliert. Man erhält bei einem Siedepunkt $Kp_{12}$ : 70-2 °C ($n_D^{20}$ : 1.456 5) 133 g ≙ 65 % d. Th. 6-Methyl-2,2,3-trifluor-1,4-benzodioxen.

19. 2,2,3-Trifluor-1,4-benzodioxen

In 600 ml Tetramethylensulfon werden 220 g Brenzkatechin und 130 g Natriumhydroxid bei 95-105 °C vorgelegt und bei dieser Temperatur unter Rühren 330 g Trifluorchloräthylen eingeleitet. Man destilliert anschließend den Ansatz bei 15 mm über eine Kolonne und fängt eine Fraktion von $Kp_{15}$ : 20 bis 60 °C in einer gut gekühlten Vorlage auf. Nachdem die $H_2O$-Phase abgetrennt wurde, verbleiben 332 g reines 2,2,3-Trifluor-1,4-benzodioxen vom Siedepunkt $Kp_{12}$ : 54-5 °C, $n_D^{20}$ : 1.452 5 mit einer Ausbeute von 87 % d. Th.

**Ansprüche**

1. Verfahren zur Herstellung der Verbindungen der Formel XIV

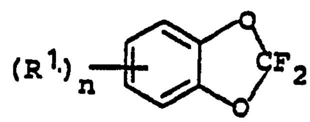

(XIV)

in welcher

n für 1 bis 4 steht und

$R^1$ für gleiche oder verschiedene Reste aus der Gruppe Wasserstoff, $C_{1-6}$-Alkyl, Trihalogenmethyl, $C_{1-6}$-Alkoxy, $C_{1-6}$-Halogenalkoxy mit 1-6 Halogenatomen, $C_{1-6}$-Alkylmercapto, $C_{1-6}$-Halogenalkylmercapto mit 1-6 Halogenatomen, für CN, —CHO, —CH—$Hal_2$, Halogen, gegebenenfalls substituiertes Phenyl oder Phenoxy, Chlorcarbonyl, Chlorsulfonyl, Nitro oder zwei benachbarte Reste gemeinsam mit den angrenzenden C-Atomen einen gegebenenfalls substituierten ankondensierten Benzolring bilden, und

$R^2$ für Wasserstoff, $C_{1-4}$-Alkyl, CN, C ≡ CH steht und

Hal für Halogen steht,

das dadurch gekennzeichnet ist, daß man Verbindungen der allgemeinen Formel XV

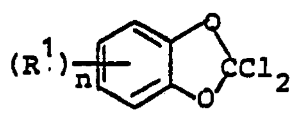

(XV)

in welcher

$R^1$ und n die oben angegebene Bedeutung haben,

mit wasserfreier Flußsäure umsetzt.

2. Verfahren zur Herstellung der Verbindungen der allgemeinen Formel XVI

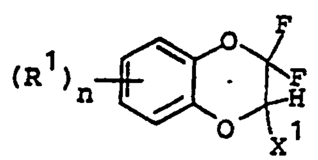

(XVI)

in welcher

n für 1 bis 4 steht und

$R^1$ für gleiche oder verschiedene Reste aus der Gruppe Wasserstoff, $C_{1-6}$-Alkyl, $C_{1-6}$-Fluoralkyl mit 3-6 Fluoratomen, $C_{1-6}$-Alkoxy, $C_{1-6}$-Halogenalkyl mit 1-6 Halogenatomen, $C_{1-6}$-Alkylmercapto, $C_{1-6}$-Halogenalkylmercapto mit 1-6 Halogenatomen, $C_{2-6}$-Alkinyl, Halogen, gegebenenfalls substituiertes Phenyl oder Phenoxy, Carbonyl, Nitro, Cyan, $SO_3H$ sowie für

$$-\underset{R^2}{\underset{|}{CH}}-OH,\ -CHO,\ -CH-Hal_2.$$

steht oder zwei der Reste einen ankondensierten Benzolring bedeuten und

$X^1$ für Wasserstoff oder Halogen steht,

$R^2$ für Wasserstoff, CN, $C_{1-4}$-Alkyl oder —C ≡ CH steht und

Hal für Halogen steht,

das dadurch gekennzeichnet ist, daß man Verbindungen der allgemeinen Formel XVII

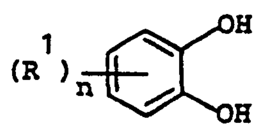

(XVII)

in welcher

$R^1$ und n die oben angegebene Bedeutung haben

mit Verbindungen der allgemeinen Formel XVIII

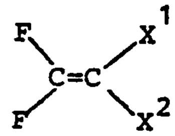

(XVIII)

in welcher

$X^2$ für Halogen steht, und

$X^1$ für Wasserstoff oder Halogen steht.

3. Verbindungen der Formel XVI

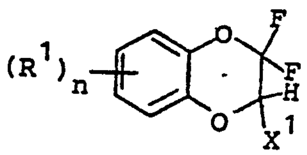

(XVI)

in welcher

n für 1 bis 4 steht und

$R^1$ für gleiche oder verschiedene Reste aus der Gruppe Wasserstoff, $C_{1-6}$-Alkyl, $C_{1-6}$-Fluoralkyl mit 3-6 Fluoratomen, $C_{1-6}$-Alkoxy, $C_{1-6}$-Halogenalkyl mit 1-6 Halogenatomen, $C_{1-6}$-Alkylmercapto, $C_{1-6}$-Halogenalkylmercapto mit 1-6 Halogenatomen, $C_{2-6}$Alkinyl, Halogen, gegebenenfalls substituiertes Phenyl oder Phenoxy, Carbonyl, Nitro, Cyan, $SO_3H$ steht oder zwei der Reste einen ankondensierten Benzolring bedeuten und

$X^1$ für Wasserstoff oder Halogen steht.

## Claims

1. Process for the preparation of compounds of the formula XIV

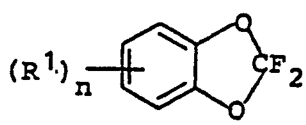

(XIV)

in which

n represents 1 to 4 and

the symbols $R^1$, for, identical or different radicals from the group comprising hydrogen, $C_{1-6}$-alkyl, trihalogenomethyl, $C_{1-6}$-alkoxy, $C_{1-6}$-halogenoalkoxy with 1-6 halogen atoms, $C_{1-6}$-alkylmercapto and $C_{1-6}$-halogenoalkylmercapto with 1-6 halogen atoms, or for CN, —CHO, —CH—$Hal_2$, halogen, optionally substituted phenyl or phenoxy, chlorocarbonyl, chlorosulphenyl or nitro, or two adjacent radicals, together with the adjoining C atoms, form an optionally substituted fused-on benzene ring and

$R^2$ represents hydrogen, $C_{1-4}$-alkyl, CN or C5 = CH and

Hal represents halogen,

which is characterised in that compounds of the general formula XV

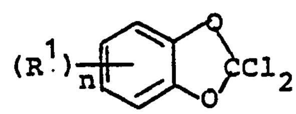

(XV)

in which

$R^1$ and n have the meaning given above, are reacted with anhydrous hydrofluoric acid.

2. Process for the preparation of compounds of the general formula XVI

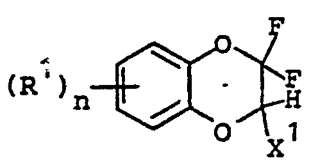

(XVI)

in which

n represents 1 to 4 and

the symbols $R^1$ represent identical or different radicals from the group comprising hydrogen, $C_{1-6}$-alkyl, $C_{1-6}$-fluoroalkyl with 3-6 fluorine atoms, $C_{1-6}$-alkoxy, $C_{1-6}$-halogenoalkyl with 1-6 halogen atoms, $C_{1-6}$-alkylmercapto, $C_{1-6}$-halogenoalkylmercapto with 1-6 halogen atoms, $C_{2-6}$-alkinyl, halogen, optionally substituted phenyl or phenoxy, carbonyl, nitro, cyano and $SO_3H$, or represent

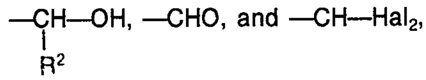

or two of the radicals denote a fused- on benzene ring and

$X^1$ represents hydrogen or halogen,

$R^2$ represents hydrogen, CN, $C_{1-4}$-alkyl or —C = CH and

Hal represents halogen,

which is characterised in that compounds of the general formula XVII

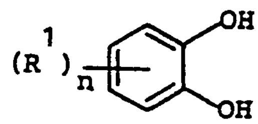

(XVII)

in which

$R^1$ and n have the meaning given above,

with compounds of the general formula XVIIII

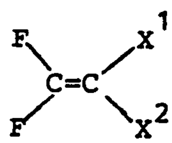

(XVIII)

in which

$X^2$ represents halogen, and

$X^1$ represents hydrogen or halogen.

3. Compounds of the formula XVI

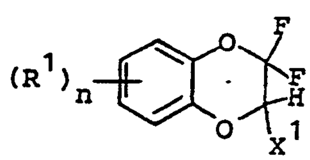

(XVI)

in which

n represents 1 to 4 and

the symbols $R^1$ represent identical of different radicals from the group comprising hydrogen, $C_{1-6}$-alkyl, $C_{1-6}$-fluoroalkyl with 3-6 fluorine atoms, $C_{1-6}$-alkoxy, $C_{1-6}$-halogenoalkyl with 1-6 halogen atoms, $C_{1-6}$-alkylmercapto, $C_{1-6}$-halogenoalkylmercapto with 1-6 halogen atoms, $C_{2-6}$-alkinyl, halogen, optionally substituted phenyl or phenoxy, carbonyl, nitro, cyano and $SO_3H$, or two of the radicals denote a fused-on benzene ring and

$X^1$ represents hydrogen or halogen.

**Revendications**

1. Procédé de production des composés de formule XIV

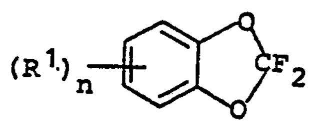

(XIV)

dans laquelle

n a une valeur de 1 à 4 et

$R^1$ représente des restes égaux ou différents choisis dans le groupe comprenant l'hydrogène, des restes alkyle en $C_1$ à $C_6$, trihalogénométhyle, alkoxy en $C_1$ à $C_6$, halogénalkoxy en $C_1$ à $C_6$ ayant 1 à 6

atomes d'halogènes, alkylmercapto en $C_1$ à $C_6$, halogénalkylmercapto en $C_1$ à $C_6$ ayant 1 à 6 atomes d'halogènes, CN, —CHO, —CH—$Hal_2$, un halogène, un reste phényle ou phénoxy éventuellement substitué, un reste chlorocarbonyle, chlorosulfonyle, nitro, ou bien deux restes voisins forment conjointement avec les atomes contigus de carbone un noyau benzénique condensé éventuellement substitué, et

$R^2$ représente l'hydrogène, un reste alkyle en $C_1$ à $C_4$, CN, C ≡ CH et

Hal représente un halogène,

qui est caractérisé en ce qu'on fait réagir des composés de formule générale XV

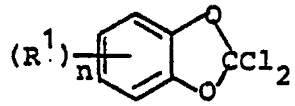

(XV)

dans laquelle

$R^1$ et n ont la définition indiquée ci-dessus,

avec l'acide fluorhydrique anhydre.

2. Procédé de production des composés de formule générale XVI

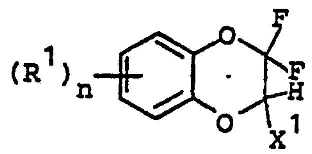

(XVI)

dans laquelle

n a une valeur de 1 à 4 et

$R^1$ représente des restes égaux ou différents choisis dans le groupe comprenant l'hydrogène, des restes alkyle en $C_1$ à $C_6$, fluoralkyle en $C_1$ à $C_6$ ayant 3 à 6 atomes de fluor, alkoxy en $C_1$ à $C_6$, halogénalkyle en $C_1$ à $C_6$ ayant 1 à 6 atomes d'halogènes, alkylmercapto en $C_1$ à $C_6$, halogénalkylmercapto en $C_1$ à $C_6$ ayant 1 à 6 atomes d'halogènes, alcynyle en $C_2$ à $C_6$, un halogène, un reste phényle ou phénoxy éventuellement substitué, un reste carbonyle, nitro, cyano, $SO_3H$ ainsi qu'un reste

—CH($R^2$)—OH, —CHO, —CH—$Hal_2$,

ou bien deux des restes forment un noyau benzénique condensé et

$X^1$ représente l'hydrogène ou un halogène,

$R^2$ est l'hydrogène, un reste —CN, un reste alkyle en $C_1$ à $C_4$ ou un reste —C ≡ CH et

Hal représente un halogène,

qui est caractérisé en ce qu'on fait (réagir) des composés de formule générale XVII

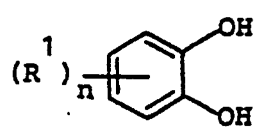

(XVII)

dans laquelle

$R^1$ et n ont la définition indiquée ci-dessus avec des composés de formule générale XVIII

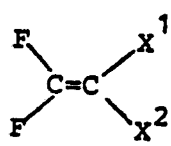

(XVIII)

dans laquelle

$X^2$ représente un halogène et

$X^1$ est l'hydrogène ou un halogène.

3. Composés de formule XVI

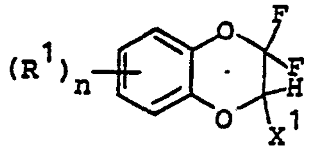

(XVI)

dans laquelle

n a une valeur de 1 à 4 et

$R^1$ représente des restes égaux ou différents choisis dans le groupe comprenant l'hydrogène, des restes alkyle en $C_1$ à $C_6$, fluoralkyle en $C_1$ à $C_6$ ayant 3 à 6 atomes de fluor, alkoxy en $C_1$ à $C_6$, halogénalkyle en $C_1$ à $C_6$ ayant 1 à 6 atomes d'halogènes, alkylmercapto en $C_1$ à $C_6$, halogénalkylmercapto en $C_1$ à $C_6$ ayant 1 à 6 atomes d'halogènes, alcynyle en $C_2$ à $C_6$, un halogène, un reste phényle ou phénoxy éventuellement substitué, un reste carbonyle, nitro, cyano, $SO_3H$, ou bien deux des restes forment un noyau benzénique condensé et

$X^1$ représente l'hydrogène ou un halogène.